# EUROPEAN PATENT APPLICATION

(11) **EP 3 795 092 A1**
(43) Date of publication of application: **24.03.2021**
(21) Application number: 19803579.2
(22) Date of filing: 17.05.2019
(51) Int. Cl.: A61B 17/06, A61B 17/34, A61F 2/00, A61B 17/00

(54) **MEDICAL SUTURE AND MEDICAL SUTURE KIT**

(30) Priority: 18.05.2018 KR 20180057110
(71) Applicant: Jworld Co., Ltd., Okcheon-gun, Chungcheongbuk-do 29055 (KR)
(72) Inventor: KANG, Min Jong, Bucheon-si, Gyeonggi-do 14613 (KR)
(74) Representative: Isarpatent
(86) International application number: PCT/KR2019/005954
(87) International publication number: WO 2019/221560

(57) **Abstract**

Embodiments of the present invention provide a medical suture which includes a body unit, a suture body, a plurality of protrusions formed on the suture body, and an anchoring cone detachably coupled to the suture body, and may have an excellent fixing force in a tissue after insertion into the body, thus to effectively perform suturing and lifting the tissue, as well as reduce a risk of infection, and a suture kit including the suture.

## Description

### [Technical Field]

The present invention relates to a suture and a suture kit, and more specifically, to a suture used for suturing and lifting a tissue and a suture kit including the suture.

### [Background Art]

A suture is used to suture an open body portion for medical purposes or to pull a sagging tissue (lifting procedure) for cosmetic purposes.

A conventional suturing method using a suture has been performed by threading a suture through a needle, then fixing an open portion by stitching, and fixing the suture by making a knot at an end of the sutured portion.

In addition, in the case of suturing by a sewing and knotting method, a difference in precision and completeness has occurred depending on a skill level and condition of an operator.

For example, according to the difference in the skill level and condition of the operator, an accident might occur, in which the open portion cannot be completely sealed, thereby resulting in death of the tissue due to suturing with an excessive force, or release of the suture since the knot comes untied.

Further, there may be a case in which the operator inevitably comes into contact with the suture during the sewing, and a pathogen penetrates into the tissue from a hand of the operator through the suture, thereby causing an infection.

Accordingly, there is a need to develop a technique to equalize the precision and completeness of the suturing and prevent an infection through the suture.

For example, Korean Patent Registration No. 10-1835763 discloses a suture including locking protrusions formed thereon, but only suturing is performed using a conventional sewing method, and does not disclose a means for preventing the infection.

### [Summary of Invention]

### [Problems to be Solved by Invention]

It is an object of the present invention to provide a suture which may be simply manufactured, and allows an operator to effectively perform suturing and lifting a tissue and a suture kit including the suture.

### [Means for Solving Problems]

To achieve the above object, the following technical solutions are adopted in the present invention.
1. A medical suture including: a suture body; a plurality of protrusions formed on the suture body; and an anchoring cone detachably coupled to the suture body.
2. The medical suture according to the above 1, wherein the protrusions and the anchoring cone are inclined in the same direction.
3. The medical suture according to the above 1, wherein the protrusions are arranged at a predetermined interval in a length direction of the suture body, and the anchoring cone is coupled to a portion of the suture body between adjacent protrusions among the protrusions.
4. The medical suture according to the above 3, wherein the protrusions are formed to face each other from both sides of the suture body to form a pair of protrusions, and the anchoring cone is coupled to a portion of the suture body between adjacent protrusion pairs among the pairs of the protrusions.
5. The medical suture according to the above 1, wherein the anchoring cone comprises an insertion groove which is formed so as to allow the suture body to be inserted, extends over an upper surface and a lower surface of the anchoring cone, and partially penetrates the anchoring cone.
6. The medical suture according to the above 5, wherein the insertion groove comprises a fastening groove with which the suture body comes into contact and fastened; and a spacer part which is communicated with the fastening groove and allows a portion of the inserted suture body to be exposed to an outside.
7. The medical suture according to the above 6, wherein a gap of the spacer part is smaller than a gap of the fastening groove.
8. The medical suture according to the above 5, wherein the anchoring cone further comprises support parts extending from the upper surface and lower surface of the anchoring cone, respectively.
9. The medical suture according to the above 1, wherein the suture body, the protrusion or the anchoring cone is made of a bioabsorbable material.
10. The medical suture according to the above 1, wherein a protrusion height of the anchoring cone from the suture body is greater than a protrusion height of the protrusion from the suture body.
11. A suture kit including: the medical suture according to any one of the above 1 to 10; and a cannula configured to house the medical suture.

### [Advantageous Effects]

According to exemplary embodiments, the medical suture may include a plurality of protrusions and an anchoring cone. The protrusions and the insertion cone are inserted into a tissue, and then are anchored and fixed to the tissue, such that a fixing force of the suture to the tissue may be improved.

For example, an anchoring cone having a large anchoring area can be anchored to the tissue, so that the tissue may be easily pulled when pulling the suture.

In addition, suturing may be performed without making a knot, and lifting may be effectively performed by pulling the suture kit.

The medical suture according to exemplary embodiments may be simply manufactured by inserting the suture body into the insertion groove of the anchoring cone.

Further, a gap between opposing walls of the fastening groove is smaller than the gap of the spacer part of the anchoring cone, such that the suture may be inserted into the fastening groove and then fixed thereto by the spacer part.

Further, it is possible to enhance a coupling force between the suture body and the anchoring cone by the support parts extending from the upper and lower surfaces of the anchoring cone, and prevent the suture from being separated during a process of inserting the same.

In exemplary embodiments, the medical suture is housed inside the cannula, thereby preventing an infection due to contact with the suture.

Furthermore, since the suture kit is made of a bioabsorbable material, a suture removal procedure may be omitted, and inflammation caused by a long-term stay of the suture in the tissue may be prevented.

### [Brief Description of Drawings]

FIG. 1 is a schematic perspective view illustrating a medical suture according to exemplary embodiments.
FIGS. 2 and 3 are schematic perspective views illustrating an anchoring cone.
FIG. 4 is a schematic perspective view illustrating a medical suture kit according to exemplary embodiments.

### [Mode for Carrying out Invention]

Embodiments of the present invention provide a medical suture which includes a body unit, a suture body, a plurality of protrusions formed on the suture body, and an anchoring cone detachably coupled to the suture body, and may have an excellent fixing force in a tissue after insertion into the body, thus to effectively perform suturing and lifting the tissue, as well as reduce a risk of infection, and a suture kit including the suture.

Hereinafter, embodiments of the present invention will be described in detail. However, these embodiments are merely an example, and the present invention is not limited to the specific embodiments described as an example. The terms "first" and "second" as used herein do not limit the number or order of objects represented by the "first" and "second", but are no more than wordings used for distinguishing the objects to be represented.

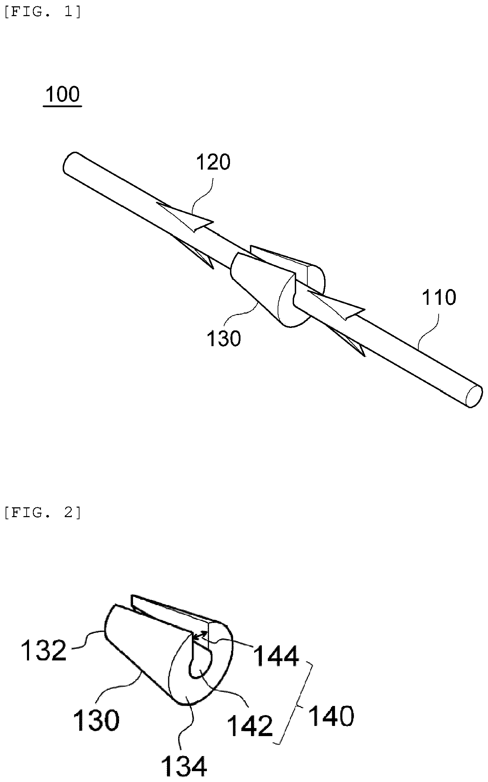

FIG. 1 is a schematic diagram illustrating a suture according to exemplary embodiments.

Referring to FIG. 1, a medical suture 100 of the present invention may include a suture body 110, protrusions 120, and an anchoring cone 130.

The suture body 110 is a body provided with the protrusions 120 of the suture and the anchoring cone 130, and may have a thread shape. The protrusions 120 and the anchoring cone 130 may be provided on an outer surface of the thread shape.

A length of the thread shape is not particularly limited, and may be formed in a length capable of sufficiently suturing the sutured portion. A thickness of the thread shape is also not particularly limited, and may be formed in a thickness capable of securing strength and fixing force without losing the flexibility of the suture body 110. For example, the suture body 110 may have a diameter of 0.3 to 3 mm.

The suture body 110 may be made of a biocompatible material. The medical suture 100 is inserted into a body tissue, and thereby, it may be formed of a material that does not cause an inflammatory reaction to various immune and antibody reactions within the body tissue.

As non-limited examples, the biocompatible material may include polypropylene, nylon, or a mixture thereof.

The suture body 110 may be made of a bioabsorbable material. The bioabsorbable material may be a material which is inserted into the body, decomposed over time, and absorbed into the tissue. Therefore, a procedure of again removing the medical suture 100 inserted into the body may be omitted. In addition, as the medical suture 100 stays in the tissue for a long time, an inflammatory reaction may occur. However, the suture body 110 of the present invention is decomposed over time, such that the inflammatory reaction may be suppressed.

In some exemplary embodiments, as non-limiting examples, the bioabsorbable material may include at least one selected from the group consisting of polydioxanone, poly(L-lactic acid), poly(glycolic acid), polycaprolactone and a copolymer thereof, or bioabsorbable metal including magnesium, zinc, and calcium, and these may be used alone or by mixing with each other.

By regulating the thickness of the bioabsorbable material and the suture body 110, a rate at which the suture body 110 is decomposed in the tissue may be controlled. For example, the decomposition rate of the suture body 110 may be controlled so that it takes several hours, several days, several months, or several years for decomposition in the tissue.

In some embodiments, the decomposition rate may be controlled by estimating the time it takes for the wound to heal after suturing. In addition, after a lifting procedure using the medical suture 100, the decomposition rate may be controlled with time to spare so that the tissue can be fixed and maintained in the lifted position.

In exemplary embodiments, the protrusions 120 may be formed along a circumference of the suture body 110. In other words, the protrusions may be formed on an outer surface of the suture body 110. In addition, a plurality of protrusions 120 may be formed thereon.

According to exemplary embodiments, the protrusion 120 may be formed to be inclined. The protrusion 120 may include a root portion in contact with the suture body 110 and a pointed portion formed at an end of the protrusion 120. For example, the pointed portion may be inclined toward one end of the suture body 110. The inclined degree (inclination angle) is not particularly limited, and it may be, for example, inclined to form a 10 to 80° angle with the suture body 110. Due to the protrusions 120 inclined toward one end, after the medical suture 100 is inserted into the tissue, the protrusions 120 penetrate between the tissues, and may be anchored in the tissue. As the protrusions 120 are anchored in the tissue, the medical suture 100 may be fixed at the inserted position. Therefore, even if a knot is not formed after suturing, the medical suture 100 may be fixed to the sutured portion.

In addition, in exemplary embodiments, after inserting the medical suture 100 into sagging tissue, when pulling the suture in a direction opposite to the insertion, it is possible to perform a lifting procedure by pulling the sagging tissue with the suture.

In some embodiments, the protrusion 120 may include a wing shape. For example, the protrusion 120 may be provided on the outer surface of the suture body 110 in a wing shape. By having the wing shape, the protrusion 120 may easily penetrate into the tissue, and may be more firmly fixed to the tissue. Therefore, the fixing force of the medical suture 100 in the tissue may be enhanced.

In exemplary embodiments, the protrusions 120 may be arranged at a predetermined interval in a length direction of the suture body 110. The interval is not particularly limited, and may range from 1 to 10 mm, for example. As the protrusions 120 are formed at the predetermined interval, the medical suture 100 may be uniformly fixed to the tissue as a whole, and thereby, the fixing force of the entire medical suture 100 may be enhanced.

In some embodiments, the protrusions 120 may be formed at opposing positions on sides of the suture body 110. For example, the protrusions 120 may be formed to face each other from both sides of the body joining body 110, thereby forming a pair of protrusions. In other words, the protrusions 120 may be formed at an angle of about 180° with respect to a center of the suture body 110. In exemplary embodiments, the protrusions 120 may be formed in several pairs, such as two pairs and three pairs. Also, for example, three protrusions 120 may be formed at an angle of 120° with respect to the center. As the protrusions 120 are formed at the above-described positions, the fixing force of the medical suture 100 may be enhanced, and when pulling the medical suture 100 during a lifting procedure using the medical suture 100, the tissue fixed by the protrusions 120 may be lifted in a uniform direction.

According to exemplary embodiments, the protrusion 120 may be made of a biocompatible material, and preferably, is made of a bioabsorbable material. As the biocompatible material and the bioabsorbable material, the same material as the material forming the suture body 110 may be used. The material forming the protrusion 120 and the material forming the suture body 110 may be the same as or different from each other. Preferably, when the protrusion 120 and the suture body 110 are made of the same material, a coupling force between the protrusion 120 and the suture body 110 may be strengthened and the decomposition rate may be balanced.

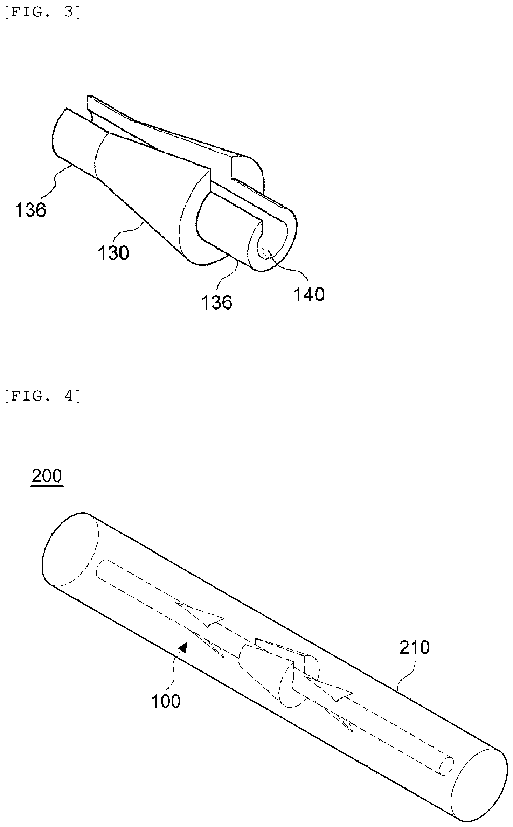

FIGS. 2 and 3 are schematic perspective views illustrating the anchoring cone. Referring to FIGS. 2 and 3, the anchoring cone 130 may include a cone upper surface 132, a cone lower surface 134, and an insertion groove 140, wherein the insertion groove 140 may include a fastening groove 142 and a spacer part 144. In addition, the anchoring cone 130 may further include support parts 136.

In some embodiments, the anchoring cone 130 may be detachably coupled to the suture body 110. The anchoring cone 130 may be anchored to the tissue after insertion into the tissue to further enhance the fixing force of the medical suture 100 in the tissue.

In exemplary embodiments, the anchoring cone 130 may be inclined in the same direction as the protrusion 120. When the anchoring cone 130 and the protrusion 120 have the same inclination direction, the anchoring cone 130 and the protrusion 120 are inserted into the tissue and then anchored in the same direction, thereby enhancing the fixing force of the medical suture.

According to exemplary embodiments, the anchoring cone 130 may be coupled to a portion between adjacent protrusions among the plurality of protrusions 120. In addition, the anchoring cone may be coupled to a suture body portion between adjacent pairs of protrusions among the pairs of the protrusions. Therefore, the anchoring of the protrusions 120 and the anchoring of the anchoring cone 130 are each independently performed, such that the fixing force of the medical suture may be enhanced.

According to some embodiments, the anchoring cone 130 may include an insertion groove 140 &which extends over the cone upper surface 132 and the cone lower surface 134 and partially penetrates the anchoring cone 130. The anchoring cone 130 may be detached from the suture body 110 through the insertion groove 140.

According to some embodiments, the cone lower surface 134 may have a larger area than the cone upper surface 132. In addition, an inclination portion may be formed on an outer periphery of the anchoring cone 130 from the cone lower surface 134 toward the cone upper surface 132. Grooves through which the insertion groove 140 passes may be formed in the cone lower surface 134 and the cone upper surface 132. Since the area of the cone lower surface 134 is wide, a lower portion of the anchoring cone 130 protrudes from the suture body 110 in a radial direction, such that the protruding portion may be anchored in the tissue. Therefore, when pulling the suture, the tissue may be easily pulled, and the suturing force and lifting effect may be improved.

In some embodiments, the anchoring cone 130 may further include the support parts 136 extending from the cone upper surface 132 and the cone lower surface 134. For example, the support parts 136 may extend from any one of the cone upper surface 132 and the cone lower surface 134, and may extend from each of the cone upper surface 132 and the cone lower surface 134.

The support part 136 has a diameter and elasticity into which the suture body 110 can be inserted. For example, the support part may be made of an elastic material with a diameter slightly smaller than the diameter of the suture body 110. When the suture body 110 is inserted into the support parts 136, the suture body 110 may be fixed to the anchoring cone 130 by elasticity and stress of the support parts 136. Therefore, by including the support parts 136, it is possible to increase the coupling force between the suture body 110 and the anchoring cone 130.

In some embodiments, the insertion groove 140 may include the fastening groove 142 and the spacer part 144. The fastening groove 142 is a portion in which the insertion groove 140 and the suture body 110 come into contact with each other to be fastened when the suture body 110 is coupled to the anchoring cone 130. The spacer part 144 has a gap so as to allow the suture body 110 to be inserted into the anchoring cone 130. The spacer part 144 is communicated with the fastening groove 142, so that the suture body 110 can pass through the spacer part 144 and be fastened to the fastening groove 142. A portion of the suture body 110 inserted through the spacer part 144 may be exposed to an outside. It is possible to easily and economically manufacture the medical suture 100 by coupling through the insertion groove 140.

In exemplary embodiments, sizes of the insertion groove 140 and the fastening groove 142 may be equal to or smaller than the diameter of the suture body 110.

According to some embodiments, the gap of the spacer part 144 may be smaller than a gap of the fastening groove 142. When the suture body 110 is inserted into the fastening groove 142, the spacer part 144 of the anchoring cone 130 covers and tightens the suture body 110, so that the anchoring cone 130 and the suture body 110 may be more strongly coupled with each other.

According to exemplary embodiments, a height of the anchoring cone 130 protruding from the suture body 110 may be greater than a protrusion height of the protrusion 120. Accordingly, the anchoring cone 130 additionally fixes a portion of the tissue which is not fixed by the protrusions 120, thereby enhancing the fixing force of the medical suture 100.

The height of the anchoring cone 130 protruding from the suture body 110 refers to a distance between a portion of the anchoring cone 130 farthest from the suture body 110 and the suture body 110 in the radial direction. For example, a portion of the cone lower surface 134 of the anchoring cone 130 may be the farthest away from the suture body 110. In addition, the protrusion height of the protrusion 120 refers to a distance between a portion of the protrusion 120 farthest from the suture body 110 and the suture body 110 in the radial direction.

According to some embodiments, the protrusion height of the anchoring cone 130 may be 5 to 20% greater than the protrusion height of the protrusion 120. In this case, the anchoring cone 130 may fix the portion of the tissue which is not fixed by the protrusion 120, as well as by appropriately adjusting the size of the anchoring cone 130, it is possible to prevent the tissue to be fixed from being destroyed, or prevent a pain from occurring in a person to be treated during the procedure.

According to exemplary embodiments, the anchoring cone 130 may be made of a biocompatible material, and preferably is made of a bioabsorbable material. As the biocompatible material and the bioabsorbable material, the same material as the material forming the suture body 110 may be used. The material forming the anchoring cone 130 and the material forming the suture body 110 may be the same as or different from each other. Preferably, when the anchoring cone 130 and the suture body 110 are made of the same material, the coupling force between the anchoring cone 130 and the suture body 110 may be strengthened and the decomposition rate may be balanced.

More preferably, by sharing the materials of the suture body 110, the protrusion 120 and the anchoring cone 130, it is possible to improve convenience during manufacturing, enhance the coupling force between the respective members, and balance the decomposition rate.

FIG. 4 is a schematic perspective view illustrating a medical suture kit according to exemplary embodiments.

Referring to FIG. 4, a medical suture kit 200 may include the medical suture 100 and a cannula 210.

According to exemplary embodiments, the cannula 210 includes holes into which medical suture 100 is inserted at both ends, through which the medical suture 100 may be inserted into the cannula 210.

Therefore, even though the medical suture 100 includes the protrusion 120 and the anchoring cone 130, it may be easily inserted into the tissue without damaging the tissue. In addition, since direct contact between the operator and the medical suture 100 is blocked during the suturing or lifting procedure, it is possible to prevent an occurrence of inflammation in the tissue due to contamination or infection of the medical suture 100.

In some embodiments, the cannula 210 may be formed to have an inner diameter of 0.4 mm to 4 mm, but it is not limited thereto, and may have any inner diameter so long as it allows the medical suture 100 to be housed. However, if the diameter exceeds 5mm, the flexibility of the cannula tube may be decreased, and excessively large holes may be formed in the tissue during suturing and lifting procedures.

In exemplary embodiments, the cannula 210 may be made of a flexible material, or may be made of a biocompatible material. As the biocompatible material, the same material as that of the suture body 110 may be used, and the same bioabsorbable material as the suture body 110 may be used. In the procedure using the suture kit 200, the cannula 210 is generally removed after the insertion, but when it is made of a bioabsorbable material, the cannula 210 may not be removed. At this time, the cannula 210 may be first decomposed, followed by the medical suture 100 being decomposed.

According to some embodiments, the cannula 210 may be a medical cannula or a medical needle, etc., which is made of a medical grade material.

According to some embodiments, the suture kit 200 may be inserted into the perforated tissue by punching or the like, and the medical suture 100 may remain in the tissue as the cannula 210 is removed after the insertion. Accordingly, the medical suture 100 including the protrusions 120 and the anchoring cone 130 may be inserted into and fixed to the tissue without hurting the tissue and without pain during the procedure.

Thereafter, by pulling the end of the medical suture 100 on a side of a direction in which the protrusions 120 and the anchoring cone 130 protrude, the opened tissue may be closed and fixed with an appropriate force. In addition, drooping and sagging tissue may be lifted and fixed. In this case, since there is no need to make a knot, the procedure may be stably performed regardless of the skill level of the operator.

## Claims

1. A medical suture comprising:
a suture body;
a plurality of protrusions formed on the suture body; and
an anchoring cone detachably coupled to the suture body.

2. The medical suture according to claim 1, wherein the protrusions and the anchoring cone are inclined in the same direction.

3. The medical suture according to claim 1, wherein the protrusions are arranged at a predetermined interval in a length direction of the suture body, and
the anchoring cone is coupled to a portion of the suture body between adjacent protrusions among the protrusions.

4. The medical suture according to claim 3, wherein the protrusions are formed to face each other from both sides of the suture body to form a pair of protrusions, and
the anchoring cone is coupled to a portion of the suture body between adjacent protrusion pairs among the pairs of the protrusions.

5. The medical suture according to claim 1, wherein the anchoring cone comprises an insertion groove which is formed so as to allow the suture body to be inserted, extends over an upper surface and a lower surface of the anchoring cone, and partially penetrates the anchoring cone.

6. The medical suture according to claim 5, wherein the insertion groove comprises a fastening groove with which the suture body comes into contact and fastened; and a spacer part which is communicated with the fastening groove and allows a portion of the inserted suture body to be exposed to an outside.

7. The medical suture according to claim 6, wherein a gap of the spacer part is smaller than a gap of the fastening groove.

8. The medical suture according to claim 5, wherein the anchoring cone further comprises support parts extending from the upper surface and lower surface of the anchoring cone, respectively.

9. The medical suture according to claim 1, wherein the suture body, the protrusion or the anchoring cone is made of a bioabsorbable material.

10. The medical suture according to claim 1, wherein a protrusion height of the anchoring cone from the suture body is greater than a protrusion height of the protrusion from the suture body.

11. A suture kit comprising:
the medical suture according to any one of claims 1 to 10; and
a cannula configured to house the medical suture.
